# EUROPÄISCHE PATENTSCHRIFT

(11) **EP 1 007 500 B1**
(45) Veröffentlichungstag und Bekanntmachung des Hinweises auf die Patenterteilung: **13.11.2002**
(21) Anmeldenummer: 98946355.9
(22) Anmeldetag: 17.08.1998
(51) Int. Cl.: C07C 67/26, C07C 69/34, C07C 69/48

(54) **VERFAHREN ZUR HERSTELLUNG VON ALKYLENGLYKOLESTERN MIT EINGESCHRÄNKTER HOMOLOGENVERTEILUNG**
METHOD FOR PRODUCING ALKYLENE GLYCOL ESTERS WITH LIMITED HOMOLOGUE DISTRIBUTION
PROCEDE DE PREPARATION D'ESTERS DE GLYCOL D'ALKYLENE A REPARTITION LIMITEE D'HOMOLOGUES

(30) Priorität: 25.08.1997 DE 19736906; 25.09.1997 DE 19741911; 23.02.1998 DE 19807597
(43) Veröffentlichungstag der Anmeldung: 14.06.2000
(73) Patentinhaber: Cognis Deutschland GmbH & Co. KG, 40589 Düsseldorf (DE)
(72) Erfinder: RATHS, Hans-Christian, D-40789 Monheim (DE)
(74) Vertreter: Reinhardt, Jürgen, Dr.
(86) Internationale Anmeldenummer: EP9805204
(87) Internationale Veröffentlichungsnummer: WO99010309

(56) Entgegenhaltungen:
- EP-A- 0 178 913
- WO-A-98/25878

## Beschreibung

Die Erfindung betrifft ein Verfahren zur Herstellung von Alkylenglykolester von unverzweigten aliphatischen Dicarbonsäuren mit eingeschränkter Homologenverteilung sowie deren Verwendung als Monomerbaustein für die Herstellung von Polymeren.

Die Anlagerung von Alkylenoxiden an CH-acide Verbindungen wie beispielsweise Fettalkohole, Alkylphenole, Fettamine oder auch Fettsäuren zählt zu den großtechnisch etablierten Verfahren zur Herstellung von nichtionischen Tensiden. Üblicherweise werden diese Reaktionen in Gegenwart homogener basischer Katalysatoren wie beispielsweise Natriumhydroxid oder Natriummethylat durchgeführt. Die Alkoxylierung stellt jedoch eine wenig selektive Reaktion dar, so daß man in der Praxis findet, daß insbesondere bei niedrigen Alkoxylierungsverhältnissen das Maximum der resultierenden Homologenverteilung nicht mit dem durchschnittlichen Alkoxylierungsgrad übereinstimmt.

Man versucht diesem unerwünschten Effekt zu begegnen, indem man Katalysatoren einsetzt, die eine höhere Selektivität aufweisen und in Summe zu Alkoxylaten, speziell Ethoxylaten, mit engerer ("eingeengter") Homologenverteilung führen; diese Produkte werden in der Literatur häufig auch als "narrow-range ethoxylates" bezeichnet. Als homogene Katalysatoren kommen für diesen Zweck vorzugsweise Erdalkalisalze, wie beispielsweise Bariumphosphat oder Strontiumethercarboxylate in Frage. Auch heterogene Katalysatoren wie beispielsweise calcinierte Hydrotalcite eignen sich hierfür.

Bezüglich der Ethoxylierung von Fettsäuren sind die Bemühungen nach dem Stand der Technik indes nach wie vor nicht befriedigend. Insbesondere wenn es darum geht, niedrig ethoxylierte Fettsäuren, speziell Fettsäure+1EO-Addukte, herzustellen, die als Vorstufen zur Synthese von Ethersulfat-Tensiden mit isethionatartiger Struktur interessant sind, werden nicht befriedigende Selektivitäten festgestellt. Neben einem unerwünschten Anteil höher ethoxylierter Homologer werden dabei insbesondere auch signifikante Mengen an Polyethylenglycol und Diestem gebildet. Auch das Verfahren gemäß dem US-Patent US 3,884,946 (Henkel), das für diesen Zweck die Verwendung von Aminen als Katalysatoren empfiehlt, liefert die niedrig ethoxylierten Fettsäuren in Ausbeuten deutlich unter 90 % der Theorie.

Gemäß der europäischen Offenlegungsschrift **EP-A-178 913** können nicht nur geradkettige Fettsäuren, sondern auch verzweigte Neocarbonsäuren mit einem tertiären Kohlenstoffatom in Nachbarstellung zur Carboxylgruppe in Gegenwart von Aminen wie Diethanolamin und Triethanolamin mit erhöhter Selektivität alkoxyliert werden. Um nach diesem Verfahren gute Ausbeuten zu erhalten, sind jedoch relativ hohe Temperaturen im Bereich von 140 bis 185 °C notwendig.

Die WO 98/25878 betrifft ein Verfahren zur Herstellung von Polyethylenglycolestern bekannt, worin einwertige Fettsäuren in Gegenwart von Alkanolaminen ethoxyliert werden. Das beschriebene Verfahren zeichnet sich durch eine besonders hohe Selektivität aus und führt insbesondere zu solchen ethoxylierten Fettsäureestem, die zwischen 0,5 und 1,5 Teile Ethylenoxid pro Molekül Fettsäure enthalten. Das Dokument macht keine konkreten Angaben darüber, dass auch Dimerfettsäuren auf diese Weise ethoxyliert werden können.

Gemäß dem zitierten Stand der Technik wurde das Problem der Selektivität bei der Alkoxylierung nur für monomere Carbonsäuren untersucht. Bislang nicht bzw. gemäß der **EP-A-178913** nur ansatzweise betrachtet wurde jedoch das Problem der selektiven Alkoxylierung bei unverzweigten aliphatischen Dicarbonsäuren. Es bestand demnach ein Bedürfnis auch für die Alkoxylierung von unverzweigten aliphatischen Dicarbonsäuren ein selektives Verfahren zur Verfügung zu stellen.

Die Aufgabe wird durch das in den Ansprüchen gekennzeichnete Verfarhren gelöst.

Im Sinne der vorliegenden Erfindung werden die Begriffe "alkoxylierte unverzweigte aliphatische Dicarbonsäuren" und Alkylenglykolester von unverzweigten aliphatischen Dicarbonsäuren° synonym gebraucht. Ebenso werden die Begriffe "Anlagerung von Alkylenoxiden "und "Alkoxylierung" synonym gebraucht.

### Dicarbonsäuren

Im Sinne der Erfindung sind unverzweigte aliphatische Dicarbonsäuren, solche, die keine Verzweigung im Kohlenwasserstoffrest tragen. Bevorzugt werden unverzweigte aliphatische alpha, omega Dicarbonsäuren der Formel **(I)**,

HOOC-R-COOH **(I)**

in der R für einen zweiwertigen, unverzweigten aliphatischen gesättigten und/oder ungesättigten Kohlenwasserstoffrest steht. Vorzugsweise steht R für einen Kohlenwasserstoffrest der beschriebenen Art mit 1 bis 20 Kohlenstoffatomen. Geeignete Dicarbonsäuren im Sinne der Erfindung sind Malonsäure, Bemsteinsäure, Adipinsäure und Azelainsäure, die kommerziell erhältlich sind, sowie die unverzweigten aliphatischen alpha, omega Dicarbonsäuren, die gemäß den deutschen Offenlegungsschriften **DE-A-37 21 119** oder **DE- A-37 38 812** durch fermentative bzw. mikrobielle Verfahren aus Alkanen, Alkenen, Alkoholen oder deren Ester in Gegenwart eines Mikroorganismus der Gattung Candida tropica lis in Anwesenheit von Nährstoffen und ggf. Co-Substraten hergestellt werden können. Nach diesen Verfahren sind besonders leicht alpha, omega unverzweigte aliphatische Dicarbonsäuren mit 10 bis 20 Kohlenwasserstoffresten (R der Formel (I)) zugänglich, die auch ungesättigt sein können.

Bevorzugt im Sinne der Erfindung werden unverzweigte aliphatische alpha, omega Dicarbonsäuren ausgewählt aus der von Malonsäure, Bernsteinsäure, Adipinsäure und Azelainsäure gebildeten Gruppe.

### Alkanolamine

Typische Beispiele für Alkanolamine, die als homogene basische Katalysatoren in Betracht kommen, sind Monoethanolamin, Diethanolamin und vorzugsweise Triethanolamin. Üblicherweise werden die Alkanolamine in Mengen von 0,05 bis 5, vorzugsweise 0,1 bis 1,5 Gew.-% - bezogen auf die Dicarbonsäuren - eingesetzt.

### Alkoxylierung

Die Alkoxylierung kann in an sich bekannter Weise durchgeführt werden und soll im folgenden exemplarisch an der Ethoxylierung beschrieben werden.

Üblicherweise legt man die unverzweigte aliphatische Dicarbonsäure und den Katalysator in einem Rührautoklaven vor, den man vor der Reaktion durch abwechselndes Evakuieren, vorzugsweise bei Temperaturen im Bereich von 80 bis 120°C, und Stickstoffspülen von Wasserspuren befreit. Anschließend wird die unverzweigte aliphatische Dicarbonsäure mit dem Ethylenoxid, welches man nach dem Aufheizen portionsweise über einen Heber in den Druckbehälter eindosieren kann, umgesetzt.

Vorzugsweise liegt das molare Umsetzungsverhältnis von unverzweigter aliphatischer Dicarbonsäure zu Ethylenoxid im Bereich von 1 : 0,5 bis 1 : 6,0, vorzugsweise 1: 1 bis 1 : 3,0. Besondere Vorteile hinsichtlich der Selektivität zeigt das Verfahren, wenn pro Mol Dicarbonsäure etwa zwei Mol Ethylenoxid umgesetzt werden (molares Einsatzverhältnis 1 :2).

Die Ethoxylierung kann bei Temperaturen im Bereich von 90°C bis 130°C durchgeführt werden. Vorzugsweise wird bei 100 bis 120°C ethoxyliert. Werden für das gesamte Verfahren Reaktionstemperaturen über 140°C gewählt, sinkt die Selektivität der Anlagerung von Ethylenoxid. Bei der Ethoxylierung empfehlen sich autogene Drücke im Bereich von 1 bis 5, vorzugsweise im Bereich von 3 bis 5, bar. Nach Reaktionsende empfiehlt es sich zur Vervollständigung des Umsatzes, eine gewisse Zeit bei der Reaktionstemperatur und den autogenen Drücken nachzurühren (15 bis 90 min). Anschließend wird der Autoklav abgekühlt, entspannt und das Produkt, falls dies gewünscht wird, mit Säuren wie z.B. Milchsäure oder Phosphorsäure versetzt, um den basischen Katalysator zu neutralisieren .

Sinngemäß gelten die obigen Ausführungen zur reinen Ethoxylierung auch für die reine Propoxylierung und für die gemischte Ethoxylierungs- und Propoxylierungsreaktion. Für die gemischte Ethoxylierungs- und Propoxylierungsreaktion kann je nach Wunsch entweder eine Mischung von Ethylenoxid und Propylenoxid oder erst Ethylenoxid und dann Propylenoxid oder umgekehrt mit den unverzweigten aliphatischen Dicarbonsäuren umgesetzt werden, wobei die molaren Einsatzmengen an unverzweigten aliphatischen Dicarbonsäuren zu Alkylenoxid, d.h. bei der gemischten Ethoxylierungs- und Propylierungsreaktion an Ethylenoxid und Propylenoxid, im oben genannten Bereich von 1 : 0,5 bis 1 : 6, vorzugsweise von 1 : 1 bis 1 : 3, liegen und insbesondere etwa 1 : 2 beträgt.

Prinzipiell ist das erfindungsgemäße Verfahren auch für die Alkoxylierung mit Butylenoxid geeignet, aber bevorzugt wird die Alkoxylierung mit Ethylenoxid und/oder Propylenoxid, insbesondere nur mit Ethylenoxid, durchgeführt.

Die Anlagerung der Alkylenoxide an die beiden Carboxylgruppen der Dicarbonsäure ist ein statistischer Prozess, d.h. mit sehr hoher Wahrscheinlichkeit lagern sich an beide Carboxylgruppen Alkylenoxid-Einheiten an.

Nach dem erfindungsgemäßen Verfahren werden Produktmischungen erhalten, wobei vorzugsweise in Mengen über 85 Gew.%, insbesondere über 90 Gew.%, Alkylenglykolmonoester der unverzweigten aliphatischen Dicarbonsäuren entstehen, die der Formel **(II)** folgen,

**H (O Alk)**_{**n**}**OOCR COO(AlkO)**_{**m**}**H (II)**

in der R die in Formel (I) wiedergegebene Bedeutung hat,
Alk für Alkylenoxidrest, insbesondere für CH₂CH₂, CHCH₃CH₂ und/oder CH₂CHCH₃ und n, m jeweils für eine Zahl im Bereich von 0 bis 6,0 steht, wobei die Summe von n+m im Bereich von 0,5 bis 6 liegt. Insbesondere steht n und m für die Zahl 1.

Wie aus Formel (II) ersichtlich, handelt es sich bei dem größten Teil der erfindungsgemäß erhaltenen Produktmischungen um Monoester von Alkylenglykolen, d.h. die angelagerten Alkylenglykole besitzen noch eine freie Hydroxylgruppe und sind an der anderen Hydroxylgruppe mit einer Carboxylgruppe der unverzweigten aliphatischen Dicarbonsäure verestert. Sofern die freie Hydroxylgruppe der Alkylenglykole mit einer weiteren Carboxylgruppe einer weiteren unverzweigten aliphatischen Dicarbonsäuren verestert ist, entstehen höher molekulare Diester von Alkylenglykolen. Ein weiterer Vorteil des erfindungsgemäßen Verfahrens liegt darin, daß der Anteil von derartigen Diestern, der bei höher molekularen unverzweigten aliphatischen Dicarbonsäuren besonders störend ist, reduziert werden konnte. So weisen die nach dem erfindungsgemäßen Verfahren hergestellten Produkte vorzugsweise einen Monoestergehalt über 85 Gew.%, insbesondere über 90 Gew.% und einen Diestergehalt unter 7 Gew.%, vorzugsweise unter 5 Gew.% auf - bezogen auf Verfahrensprodukt -. Der zu 100 Gew.% fehlende Rest stellt nicht umgesetzte Restsäure dar.

Die Selektivität des erfindungsgemäßen Verfahrens ist daran ersichtlich, daß mindestens 90 Gew.%, vorzugsweise mindestens 95 Gew.%, der Verbindungen nach Formel (II) als Alkoxylierungsgrad n bzw. m etwa die gleiche Zahl aufweisen, wobei die Summe von n und m den Umsetzungsverhältnissen an Alkylenoxid pro Mol unverzweigter aliphatischer Dicarbonsäure entsprechen. Mit anderen Worten, entstehen bei einer Anlagerung von 2 Mol Ethylenoxid an 1 Mol unverzweigter aliphatischer Dicarbonsäuren Verbindungen der Formel (II), die mindestens zu 90 Gew.% für n und m etwa die gleiche Zahl (d.h. 1) aufweisen.

Die nach dem erfindungsgemäßen Verfahren erhaltenen Anlagerungsprodukte können im weitesten Sinne als Diole mit Esterfunktion verstanden werden, die beispielsweise bei der Herstellung von Polyestern verwendet werden können. Ein weiterer Gegenstand der vorliegenden Erfindung betrifft daher die Verwendung der Anlagerungsprodukte von Alkylenoxiden an unverzweigte aliphatische Dicarbonsäuren hergestellt nach Anspruch 1 als Monomerbaustein für Polymere, insbesondere für Polyester.

### Beispiele

### Beispiel 1:

In einem Autoklaven wurden 658,5 g (3,5 Mol) Azelainsäure) vorgelegt und mit 7,3 g Triethanolamin (entsprechend 1,2 Gew.-% bezogen auf Dicarbonsäure) versetzt. Der Autoklav wurde dreimal abwechselnd 30 Minuten bei 80°C und 30 mbar evakuiert und mit Stickstoff beaufschlagt, um Spuren von Wasser, die zur Bildung von Polyethylenglycol führen könnten, zu entfemen. Nachdem die Reaktionsmischung ein letztesmal mit Stickstoff beaufschlagt worden war, wurde der Autoklav verschlossen auf 100°C erhitzt und portionsweise bei einem Maximaldruck von 5 bar mit 308 g (7 Mol) Ethylenoxid beaufschlagt. Nach Abschluß der Reaktion, erkenntlich dadurch, daß der Druck wieder bis auf einen Wert von 1,2 bar abfiel und dann konstant blieb, wurde 60 min nachgerührt bei 100 °C und 5 bar und der Reaktionsansatz anschließend abgekühlt und entspannt. Der basische Katalysator verblieb im Endprodukt.

Es wurde ein Produkt erhalten, welches einen Monoestergehalt mit 1 Mol Ethylenoxid pro Carboxylgruppe von 91,3 Gew.-%, einen Monestergehalt mit mehr als 1 Mol Ethylenoxid pro Carboxylgruppe von 2,6 Gew.-%, einen Ethylenglykoldiestergehalt von 3,0 Gew.-% und einen Restsäuregehalt von 3,1 Gew.-% aufwies.

Die Zusammensetzung des Produktes zeigt, daß zum einen das erfindungsgemäße Verfahren in hohen Ausbeuten Monoester erzeugt und zum anderen, daß das erfindungsgemäße Verfahren äußerst selektiv ist, da die überwiegende Menge der Monoester Verbindungen sind, die pro Mol Carboxylgruppe der unverzweigten aliphatischen Dicarbonsäure nur 1 Mol Ethylenoxid - wie nach den eingesetzten Mengen an Ethylenoxid gewünscht - aufweisen.

## Patentansprüche

1. Verfahren zur Herstellung von Monoalkylenglycolestem aliphatischer Dicarbonsäuren durch Umsetzung der Dicarbonsäuren und Alkylenoxiden in Gegenwart von Alkanolaminen als basische Katalysatoren, **dadurch gekennzeichnet, dass** unverzweigte aliphatische Dicarbonsäuren umgesetzt werden.

2. Verfahren nach Anspruch 1, **dadurch gekennzeichnet, daß** unverzweigte aliphatische alpha, omega Dicarbonsäuren der Formel **(I)**,
HOOC-R-COOH **(I)**
in der R für einen zweiwertigen, unverzweigten aliphatischen gesättigten und/oder ungesättigten Kohlenwasserstoffrest steht, eingesetzt werden.

3. Verfahren nach einem der Ansprüche 1 oder 2, **dadurch gekennzeichnet, daß** unverzweigte aliphatische alpha, omega Dicarbonsäuren ausgewählt aus der von Malonsäure, Bernsteinsäure, Adipinsäure und Azelainsäure gebildeten Gruppe eingesetzt werden.

4. Verfahren nach Anspruch 1, **dadurch gekennzeichnet, daß** Ethylenoxid und/oder Propylenoxid, vorzugsweise nur Ethylenoxid, an die unverzweigten alipahtischen Dicarbonsäuren angelagert werden.

5. Verfahren nach Anspruch 1, **dadurch gekennzeichnet, daß** die unverzweigten aliphatischen Dicarbonsäuren mit den Alkylenoxiden im molaren Verhältnis von 1: 0,5 bis 1 : 6, vorzugsweise 1 : 1 bis 1 : 3, umgesetzt werden.

6. Verfahren nach Anspruch 1, **dadurch gekennzeichnet, daß** als Alkanolamin Triethanolamin eingesetzt wird.

7. Verfahren nach Anspruch 1, **dadurch gekennzeichnet, daß** die Alkanolamine in Mengen von 0,05 bis 5 Gew.% - bezogen auf Dicarbonsäuren - eingesetzt werden.

8. Verfahren nach Anspruch 1, **dadurch gekennzeichnet, daß** die Anlagerung der Alkylenoxide an die unverzweigten aliphatischen Dicarbonsäuren bei Temperaturen im Bereich von 90 bis 130 °C durchgeführt wird.

9. Verfahren nach Anspruch 1, **dadurch gekennzeichnet, daß** die Anlagerung der Alkylenoxide an die unverzweigten aliphatischen Dicarbonsäuren bei autogenen Drücken im Bereich von 1 bis 5 bar, vorzugsweise von 3 bis 5 bar, durchgeführt wird.

10. Verwendung der Anlagerungsprodukte von Alkylenoxiden an unverzweigte aliphatische Dicarbonsäuren hergestellt nach Anspruch 1 als Monomerbaustein für Polymere, insbesondere für Polyester.

## Claims

1. A process for the production of monoalkylene glycol esters of aliphatic dicarboxylic acids by reaction of the dicarboxylic acids and alkylene oxides in the presence of alkanolamines as basic catalysts, **characterized in that** unbranched aliphatic dicarboxylic acids are reacted.

2. A process as claimed in claim 1, **characterized in that** unbranched aliphatic α,ω-dicarboxylic acids corresponding to formula **(I)**:
**HOOC-R-COOH (I)**
in which R is a difunctional, unbranched, aliphatic, saturated and/or unsaturated hydrocarbon radical, are used.

3. A process as claimed in claim 1 or 2, **characterized in that** unbranched aliphatic α,ω-dicarboxylic acids selected from the group consisting of malonic acid, succinic acid, adipic acid and azelaic acid are used.

4. A process as claimed in claim 1, **characterized in that** ethylene oxide and/or propylene oxide, preferably ethylene oxide only, is/are added onto the unbranched aliphatic dicarboxylic acids.

5. A process as claimed in claim 1, **characterized in that** the unbranched aliphatic dicarboxylic acids are reacted with the alkylene oxides in a molar ratio of 1:0.5 to 1:6 and preferably in a molar ratio of 1:1 to 1:3.

6. A process as claimed in claim 1, **characterized in that** triethanolamine is used as the alkanolamine.

7. A process as claimed in claim 1, **characterized in that** the alkanolamines are used in quantities of 0.05 to 5% by weight, based on dicarboxylic acids.

8. A process as claimed in claim 1, **characterized in that** the addition of the alkylene oxides onto the unbranched aliphatic dicarboxylic acids is carried out at temperatures of 90 to 130°C.

9. A process as claimed in claim 1, **characterized in that** the addition of the alkylene oxides onto the unbranched aliphatic dicarboxylic acids is carried out under autogenous pressures of 1 to 5 bar and preferably 3 to 5 bar.

10. The use of the addition products of alkylene oxides with unbranched aliphatic dicarboxylic acids produced by the process claimed in claim 1 as a monomer unit for polymers, more particularly for polyesters.

## Revendications

1. Procédé de préparation d'esters de monoalkylèneglycols d'acides dicarboxyliques aliphatiques par réaction des acides dicarboxyliques et des oxydes d'alkylène en présence d'alkanolamines en tant que catalyseurs basiques,
**caractérisé en ce qu'**on fait réagir des acides dicarboxyliques aliphatiques non ramifiés.

2. Procédé selon la revendication 1,
**caractérisé en ce qu'**
on met en oeuvre des acides dicarboxyliques en alpha-oméga aliphatiques non ramifiés de formule **(I)**,
HOOC-R-COOH **(I)**
dans laquelle R représente un reste hydrocarboné saturé et/ou non saturé aliphatique non ramifié,. difonctionnel,

3. procédé selon l'une quelconque des revendications 1 ou 2,
**caractérisé en ce qu'**
on met en oeuvre des acides dicarboxyliques en alpha-oméga, aliphatiques non ramifiés choisis dans le groupe formé par l'acide malonique, l'acide succinique, l'acide adipique, et l'acide azélaïque.

4. Procédé selon la revendication 1,
**caractérisé en ce qu'**
on additionne de l'oxyde d'éthylène et/ou de l'oxyde de propylène, de préférence seulement de l'oxyde d'éthylène sur les acides dicarboxyliques aliphatiques non ramifiés.

5. Procédé selon la revendication 1,
**caractérisé en ce qu'**
on fait réagir les acides dicarboxyliques aliphatiques non ramifiés avec les oxydes d'alkylène dans un rapport molaire allant de 1:0,5 à 1:6, de préférence de 1:1 à 1:3.

6. Procédé selon la revendication 1,
**caractérisé en ce qu'**
on met en oeuvre comme alkanolamine, la triéthanolamine.

7. Procédé selon la revendication 1,
**caractérisé en ce qu'**
on met en oeuvre les alkanolamines en quantités allant de 0,05 à 5 % en poids par rapport aux acides dicarboxyliques.

8. Procédé selon la revendication 1,
**caractérisé en ce qu'**
on effectue l'addition de l'oxyde d'alkylène sur les acides dicarboxyliques aliphatiques non ramifiés à des températures dans la zone de 90 à 130°C.

9. Procédé selon la revendication 1,
**caractérisé en ce qu'**
on effectue l'addition des oxydes d'alkylène sur les acides dicarboxyliques aliphatiques non ramifiés à des pressions autogènes dans la zone de 1 à 5 bars, de préférence de 3 à 5 bars.

10. Utilisation des produits d'addition des oxydes d'alkylène sur des acides dicarboxyliques aliphatiques non ramifiés, préparés selon la revendication 1, en tant qu'éléments constitutifs monomères pour des polymères, en particulier pour des polyesters.
